# EUROPEAN PATENT SPECIFICATION

(11) **EP 0 817 643 B1**
(45) Date of publication and mention of the grant of the patent: **21.03.2007**
(21) Application number: 96913778.5
(22) Date of filing: 01.04.1996
(51) Int. Cl.: A61K 38/00, A61K 47/12, C07C 53/126, C07C 53/134, C07C 57/26, C07C 57/30, C07C 59/76, C07C 59/205, C07C 229/10, C07C 311/19, C07D 207/34

(54) **COMPOUNDS AND COMPOSITIONS FOR DELIVERING ACTIVE AGENTS**
STOFFE UND ZUSAMMENSETZUNGEN FÜR DIE VERABREICHUNG AKTIVER SUBSTANZEN
COMPOSES ET COMPOSITIONS SERVANT A L'APPORT D'AGENTS ACTIFS

(30) Priority: 31.03.1995 US 414654; 01.09.1995 US 3111 P; 29.03.1996 US 17902 P
(43) Date of publication of application: 14.01.1998
(73) Proprietor: Emisphere Technologies, Inc., Tarrytown, New York 10591-6715 (US)
(72) Inventor: LEONE-BAY, Andrea, Ridgefield, CT 06877 (US); HO, Koc-Kan, Mt. Kisco, NY 10549 (US); SARUBBI, Donald, J., Bronxville, NY 10708 (US); MILSTEIN, Sam, J., Larchmont, NY 10538 (US); PRESS, Jeffery, Bruce, Brewster, NY 10509 (US)
(74) Representative: Grünecker, Kinkeldey, Stockmair & Schwanhäusser Anwaltssozietät
(86) International application number: PCT/US1996/004580
(87) International publication number: WO 1996/030036

(56) References cited:
- WO-A-94/23767
- WO-A-95/28838
- WO-A-95/28920
- US-A- 3 489 793
- US-A- 3 931 153
- US-A- 4 613 618
- US-A- 4 757 066
- US-A- 5 451 410
- MILSTEIN S.: "Oral bioavailability of partially folded proteins" PROCEEDINGS OF THE 1995 MIAMI BIO/TECHNOLOGY WINTER SYMPOSIUM. ADVANCES IN GENE TECHNOLOGY: PROTEIN ENGINEERING AND STRUCTURAL BIOLOGY. SHORT REPORTS, vol. 6, 4 - 9 February 1995, page 13 XP002287021
- IBRAHIM EL S A ET AL: "SYNTHESIS OF 4-SUBSTITUTED AMINOBENZOATE QUATERNARY SALTS AS POTENT ANTISPASMODIC AGENTS" JOURNAL OF PHARMACEUTICAL SCIENCES, AMERICAN PHARMACEUTICAL ASSOCIATION. WASHINGTON, US, vol. 68, no. 3, March 1979 (1979-03), pages 332-335, XP001085297 ISSN: 0022-3549
- PICCIOLA G.: "Sintesi di acidi china- zolinonici e benzossazinonici e studio delle loro proprieta antiinfiammatorie" FARMACO, EDIZIONE SCIENTIFICA., vol. 31, no. 9, 1976, pages 655-664, XP002286599 ITSOCIETA CHIMICA ITALIANA, PAVIA.
- JORGENSEN E.C. ET AL.: "Angiotensin II analogs. IV. Synthesis and biological evaluation of simplified angiotensins" JOURNAL OF MEDICINAL CHEMISTRY., vol. 13, no. 4, 1970, pages 744-745, XP002286600 USAMERICAN CHEMICAL SOCIETY. WASHINGTON.
- LEONE-BAY A. ET AL.: "N-acylated alpha-amino acids as novel oral delivery agents for proteins" JOURNAL OF MEDICINAL CHEMISTRY., vol. 38, no. 21, 13 October 1995 (1995-10-13), pages 4263-4269, XP002286601 USAMERICAN CHEMICAL SOCIETY. WASHINGTON.
- MORRISON et al., "Organic Chemistry", Third Edition, BOSTON: ALLYN AND BACON, INC., December 1973, pages 671, 672, 755-758. XP002903333

## Description

### FIELD OF THE INVENTION

The present invention relates to compounds for delivering active agents, and particularly biologically or chemically active agents such as, for example, bioactive peptides and the like. These compounds are used as carriers to facilitate the delivery of a cargo to a target. The carriers are modified amino acids and are well suited to form non-covalent mixtures with biologically-active agents for oral administration to animals. Methods for the preparation and for the administration of such compositions are also disclosed.

### BACKGROUND OF THE INVENTION

Conventional means for delivering active agents are often severely limited by biological, chemical, and physical barriers. Typically, these barriers are imposed by the environment through which delivery occurs, the environment of the target for delivery, or the target itself.

Biologically or chemically active agents are particularly vulnerable to such barriers. For example in the delivery to animals of pharmacological and therapeutic agents, barriers are imposed by the body. Examples of physical barriers are the skin and various organ membranes that must be traversed before reaching a target. Chemical barriers include, but are not limited to, pH variations, lipid bi-layers, and degrading enzymes.

These barriers are of particular significance in the design of oral delivery systems. Oral delivery of many biologically or chemically active agents would be the route of choice for administration to animals if not for biological, chemical, and physical barriers such as varying pH in the gastro-intestinal (GI) tract, powerful digestive enzymes, and active agent impermeable gastro-intestinal membranes. Among the numerous agents which are not typically amenable to oral administration are biologically or chemically active peptides, such as calcitonin and insulin; polysaccharides, and in particular mucopolysaccharides including, but not limited to, heparin; heparinoids; antibiotics; and other organic substances. These agents are rapidly rendered ineffective or are destroyed in the gastro-intestinal tract by acid hydrolysis, enzymes, or the like.

Earlier methods for orally administering vulnerable pharmacological agents have relied on the co-administration of adjuvants (e.g., resorcinols and non-ionic surfactants such as polyoxyethylene oleyl ether and n-hexadecylpolyethylene ether) to increase artificially the permeability of the intestinal walls, as well as the co-administration of enzymatic inhibitors (e.g., pancreatic trypsin inhibitors, diisopropylfluorophosphate (DFF) and trasylol) to inhibit enzymatic degradation.

Liposomes have also been described as drug delivery systems for insulin and heparin. See, for example, U.S. Patent No. 4,239,754; Patel et al. (1976), *FEBS Letters,* Vol. 62, pg. 60; and Hashimoto et al. (1979), *Endocrinology Japan,* Vol. 26, pg. 337.

However, broad spectrum use of such drug delivery systems is precluded because: (1) the systems require toxic amounts of adjuvants or inhibitors; (2) suitable low molecular weight cargos, i.e. active agents, are not available; (3) the systems exhibit poor stability and inadequate shelf life; (4) the systems are difficult to manufacture; (5) the systems fail to protect the active agent (cargo); (6) the systems adversely alter the active agent; or (7) the systems fail to allow or promote absorption of the active agent.

More recently, microspheres of artificial polymers of mixed amino acids (proteinoids) have been used to deliver pharmaceuticals. For example, U.S. Patent No. 4,925,673 describes drug-containing proteinoid microsphere carriers as well as methods for their preparation and use. These proteinoid microspheres are useful for the delivery of a number of active agents.

There is still a need in the art for simple, inexpensive delivery systems which are easily prepared and which can deliver a broad range of active agents.

### SUMMARY OF THE INVENTION

Compositions which are useful in the delivery of active agents are provided. These compositions include at least one active agent, and preferably a biologically or chemically active agent, and at least one of the following compounds 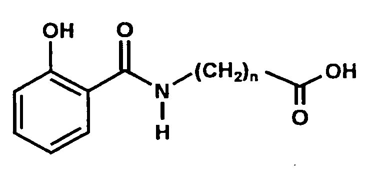 wherein

| Compound | n |
|---|---|
| IV | 3 |
| XIX | 7 |
| XXVII | 10 |
| XXVIII | 8 |
| XXIX | 6 |
| XXX | 11 |
| XXXI | 9 |

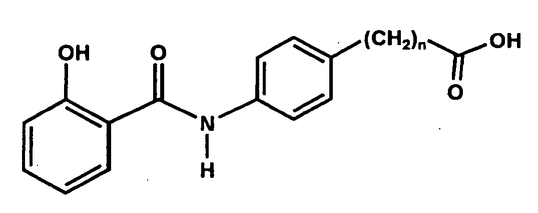

wherein

| | |
|---|---|
| Compound | n |
| LII | 1 |
| LIV | 2 |

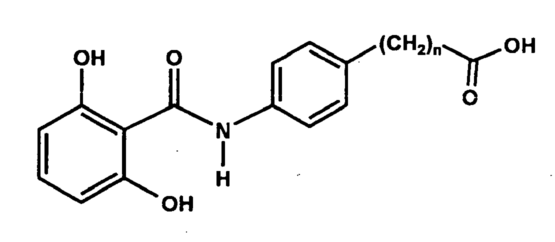

wherein

| | |
|---|---|
| Compound | n |
| Llll | 3 |
| LVI | 2 |

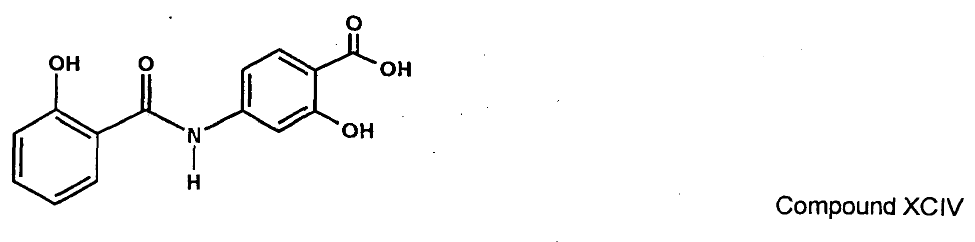

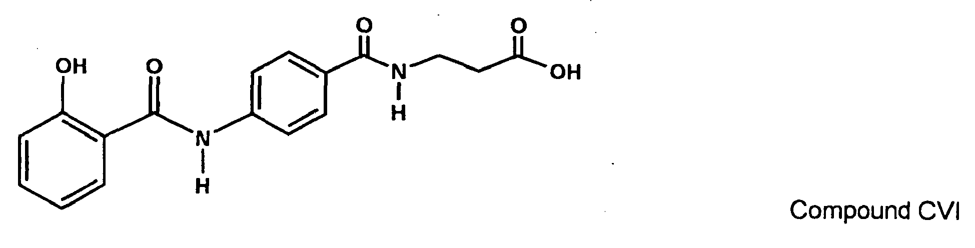

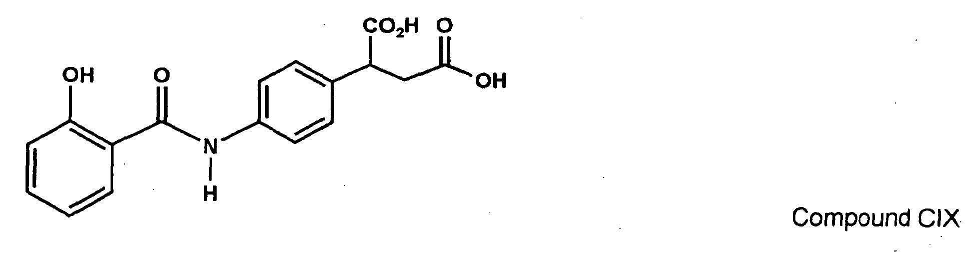

or salts thereof.

It has been discovered that organic acid compounds, and their salts, having an aromatic amide group, having a hydroxy group substituted in the ortho position on the aromatic ring, and a lipophilic chain with from about 4 carbon atoms to about 20 atoms in the chain are useful as carriers for the delivery of active agents. In a preferred form the lipophilic chain can have from 5 to 20 carbon atoms.

Compositions comprising the carrier compounds discussed above and active agents have been shown effective in delivering active agents to selected biological systems. These compositions include at least one active agent which is preferably a biologically or chemically active agent, and at least one carrier compound.

Further contemplated by the present invention are dosage unit forms that include these compositions.

Also contemplated is a method for preparing these compositions which comprises mixing at least one active agent with at least one compound as described above, and optionally, a dosing vehicle.

In an alternative embodiment, these non-toxic compounds are orally administered to animals as part of a delivery system by blending or mixing the compounds with an active agent prior to administration.

### DESCRIPTION OF THE DRAWINGS

Figure 1 is a graphic illustration of the results of subcutaneous injection of rhGH composition in rats.
Figure 2 is a graphic illustration of the results of Sublingual (SL), intranasal (IN), and intracolonic (IC) dosing of rhGH in rats.

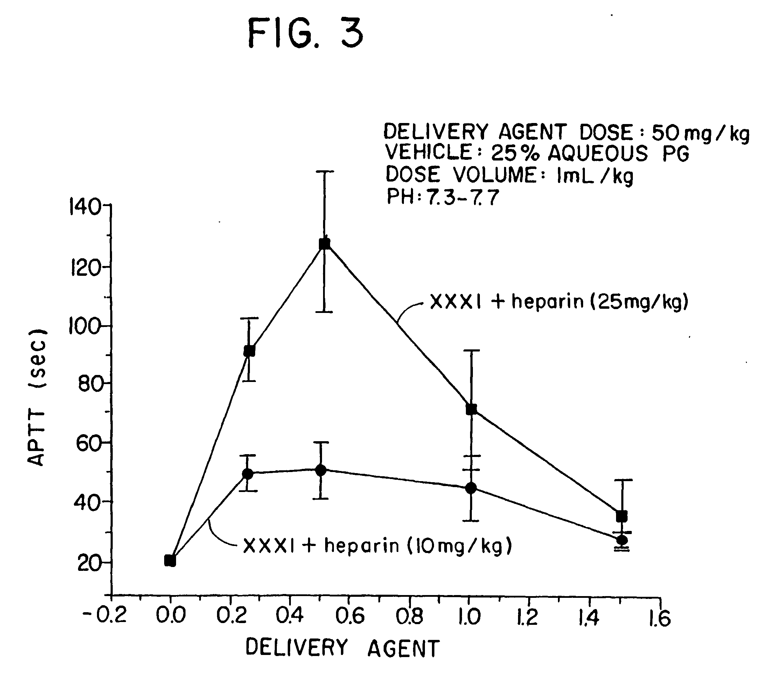

Figure 3 is a graphic illustration of the results of intracolonic dosing of delivery of heparin with compound XXXI carrier.

### DETAILED DESCRIPTION OF THE INVENTION

The specific compositions of the present invention include an active agent and a modified amino acid. These compositions may be used to deliver various active agents through various biological, chemical, and physical barriers and are particularly suited for delivering active agents which are subject to environmental degradation. The compositions of the subject invention are particularly useful for delivering or administering biologically or chemically active agents to any animals such as birds; mammals, such as primates and particularly humans; and insects.

Other advantages of the present invention include the use of easy to prepare, inexpensive raw materials. The compositions and the formulation methods of the present invention are cost effective, simple to perform, and amenable to industrial scale up for commercial production.

Subcutaneous, sublingual, and intranasal coadministration of an active agent, such as recombinant human growth hormone (rhGH), and the delivery agents, and particularly proteins, described herein results in an increased bioavailability of the active agent compared to administration of the active agent alone. A similar result is obtained by coadministration of salmon calcitonin with the delivery agents, in rats. Data supporting these findings are presented in the examples.

### Active Agents

Active agents suitable for use in the present invention include biologically or chemically active agents, chemically active agents, including, but not limited to, fragrances, as well as other active agents such as, for example, cosmetics.

Biologically or chemically active agents include, but are not-limited to, pesticides, pharmacological agents, and therapeutic agents. For example, biologically or chemically active agents suitable for use in the present invention include, but are not limited to, peptides, and particularly small peptides; hormones, and particularly hormones which by themselves do not or only a fraction of the administered dose passes through the gastro-intestinal mucosa and/or are susceptible to chemical cleavage by acids and enzymes in the gastro-intestinal tract; polysaccharides, and particularly mixtures of muco-polysaccharides; carbohydrates; lipids; or any combination thereof. Further examples include, but are not limited to, human growth hormones; bovine growth hormones; growth releasing hormones; interferons; interleukin-1; insulin; heparin, and particularly low molecular weight heparin; calcitonin; erythropoietin; atrial naturetic factor; antigens; monoclonal antibodies; somatostatin; adrenocorticotropin, gonadotropin releasing hormone; oxytocin; vasopressin; cromolyn sodium (sodium or disodium chromoglycate); vancomycin; desferrioxamine (DFO); parathyroid hormone anti-microbials, including, but not limited to anti-fungal agents; or any combination thereof.

### Modified Amino Acids

The term modified amino acid is used to designate the above mentioned carrier compounds which are amino acids which have be en modified by acylating a free amine group with an acylating agent which reacts with the free amine group present.

Amino acids in modified form, may be used to deliver active agents including, but not limited to, biologically or chemically active agents such as for example, pharmacological and therapeutic agents.

Modified amino acids are typically prepared by modifying the amino acid or an ester thereof. Many of these compounds are prepared by acylation with agents having the formula

X - Y - R⁴

wherein:
R⁴ is the appropriate radical to yield the modification indicated in the final product,
Y is 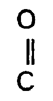 and X is a leaving group. Typical leaving groups include, but are not limited to, halogens such as, for example, chlorine, bromine, and iodine. Additionally, the corresponding anhydrides are modifying agents.

Many of the compounds of the present invention can be readily prepared from amino acids by methods within the skill of those in the art based upon the present disclosure. For example, compounds I-VII are derived from aminobutyric acid; and Compounds XI-XXVI are derived from aminocaprylic acid. For example, the modified amino acid compounds above may be prepared by reacting the single amino acid with the appropriate modifying agent which reacts with free amino moiety present in the amino acids to form amides. Protecting groups may be used to avoid unwanted side reactions as would be known to those skilled in the art.

The amino acid can be dissolved in aqueous alkaline solution of a metal hydroxide, e.g., sodium or potassium hydroxide, and heated at a temperature ranging between about 5°C and about 70°C, preferably between about 10°C and about 40°C, for a period ranging between about 1 hour and about 4 hours, preferably about 2.5 hours. The amount of alkali employed per equivalent of NH₂ groups in the amino acid generally ranges between about 1.25 and about 3 mmole, preferably between about 1.5 and about 2.25 mmole per equivalent of NH₂. The pH of the solution generally ranges between about 8 and about 13, preferably ranging between about 10 and about 12.

Thereafter, the appropriate amino modifying agent is added to the amino acid solution while stirring. The temperature of the mixture is maintained at a temperature generally ranging between about 5 ° C and about 70°C, preferably between about 10°C and about 40°C, for a period ranging between about 1 and about 4 hours. The amount of amino modifying agent employed in relation to the quantity of amino acid is based on the moles of total free NH₂ in the amino acid. In general, the amino modifying agent is employed in an amount ranging between about 0.5 and about 2.5 mole equivalents, preferably between about 0.75 and about 1.25 equivalents, per molar equivalent of total NH₂ group in the amino acid.

The reaction is quenched by adjusting the pH of the mixture with a suitable acid, e.g., concentrated hydrochloric acid, until the pH reaches between about 2 and about 3. The mixture separates on standing at room temperature to form a transparent upper layer and a white or off-white precipitate. The upper layer is discarded, and the modified amino acid is collected from the lower layer by filtration or decantation. The crude modified amino acid is then dissolved in water at a pH ranging between about 9 and about 13, preferably between about 11 and about 13. Insoluble materials are removed by filtration and the filtrate is dried in vacuo. The yield of modified amino acid generally ranges between about 30 and about 60%, and usually about 45%.

If desired, amino acid esters, such as, for example benzyl, methyl, or ethyl esters of amino acid compounds, may be used to prepare the modified amino acids of the invention. The amino acid ester, dissolved in a suitable organic solvent such as dimethylformamide, pyridine, or tetrahydrofuran is reacted with the appropriate amino modifying agent at a temperature ranging between about 5°C and about 70°C, preferably about 25°C, for a period ranging between about 7 and about 24 hours. The amount of amino modifying agent used relative to the amino acid ester is the same as described above for amino acids. This reaction may be carried out with or without a base such as, for example, triethylamine or diisopropylethylamine.

Thereafter, the reaction solvent is removed under negative pressure and the ester functionality is removed by hydrolyzing the modified amino acid ester with a suitable alkaline solution, e.g. 1N sodium hydroxide, at a temperature ranging between about 50°C and about 80°C, preferably about 70°C, for a period of time sufficient to hydrolyze off the ester group and form the modified amino acid having a free carboxyl group. The hydrolysis mixture is then cocled to room temperature and acidified, e.g. aqueous 25% hydrochloric acid solution, to a pH ranging between about 2 and about 2.5. The modified amino acid precipitates out of solution and is recovered by conventional means such as filtration or decantation. Benzyl esters may be removed by hydrogenation in an organic solvent using a transition metal catalyst.

The modified amino acid may be purified by recrystallization or by fractionation on solid column supports. Suitable recrystallization solvent systems include acetonitrile, methanol and tetrahydrofuran. Fractionation may be performed on a suitable solid column supports such as alumina, using methanol/n-propanol mixtures as the mobile phase; reverse phase column supports using trifluoroacetic acid/acetonitrile mixtures as the mobile phase; and ion exchange chromatography using water as the mobile phase. When anion exchange chromatography is performed, preferably a subsequent 0-500 mM sodium chloride gradient is employed.

In an alternate method modified amino acids having the formula 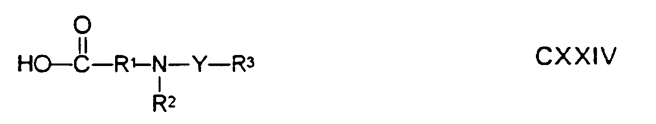 wherein Y is 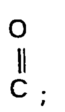 and R¹, R² and R³ are the appropriate radicals ; may be prepared by
(a) reacting in water and the presence of a base a compound having the formula 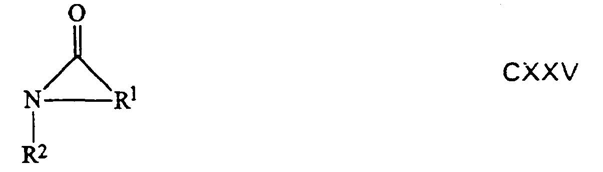 with a compound having the formula

   R³-Y-X,

   wherein
   Y, R¹, R², and R³ are as above and X is a leaving group.

Compound CXXV can be prepared, for example by the method described in Olah et al., Synthesis, 537-538 (1979).

Compound XXXI was prepared as described in Scheme I from 10-undecen-1-ol, 1, by a three step procedure in an overall yield of 31 %. Alkylation of phthalimide with alkanol, 1, under Mitsunobu conditions, followed by reaction with hydrazine gave 1-aminoundec-10-ene, 2, in 66% yield. The amine was derivatized with *O*-acetylsalicyloyl chloride and the resulting alkene, 3, was oxidized to the acid using potassium permanganate. Removal of the acetate, followed by acid precipitation provided compound XXXI in 47% yield based on amine 2.

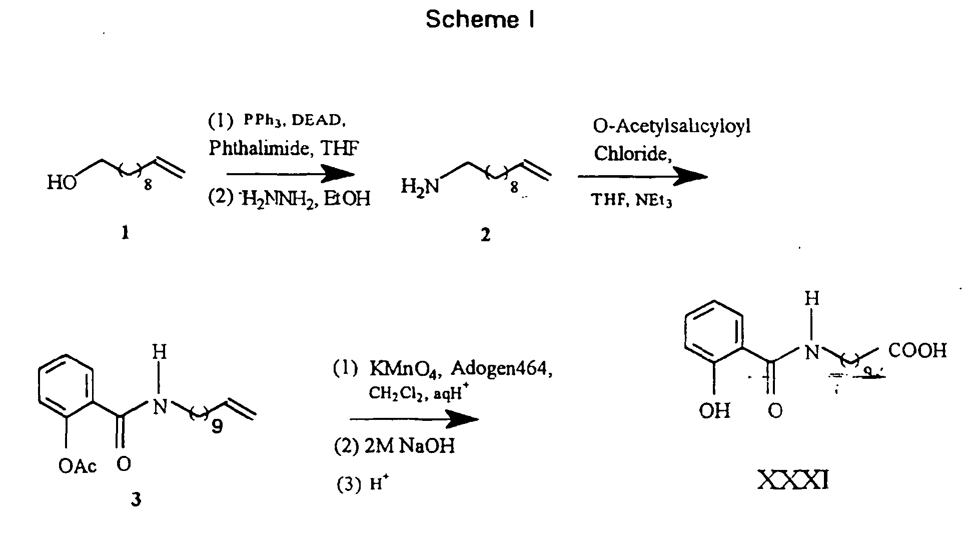

### Delivery Systems

The compositions of the present invention may include one or more active agents.

In one embodiment, the above mentioned carrier compounds may be used directly as a delivery carrier by simply mixing one or more compound with the active agent prior to administration.

In an alternative embodiment, the compounds may be used to form microspheres containing the active agent. These compounds are particularly useful for the oral administration of certain biologically-active agents, *e.g.,* small peptide hormones, which, by themselves, do not pass or only a fraction of the administered dose passes through the gastro-intestinal mucosa and/or are susceptible to chemical cleavage by acids and enzymes in the gastrointestinal tract.

If the modified amino acids are to be converted into microspheres, the mixture is optionally heated to a temperature ranging between about 20 and about 50°C, preferably about 40°C, until the modified amino acid(s) dissolve. The final solution contains between from about 1 mg and to about 2000 mg of compound per mL of solution, preferably between about 1 and about 500 mg per mL. The concentration of active agent in the final solution varies and is dependent on the required dosage for treatment. When necessary, the exact concentration can be determined by, for example, reverse phase HPLC analysis.

When the compounds are used to prepare microspheres, another useful procedure is as follows: Compounds are dissolved in deionized water at a concentration ranging between about 75 and about 200 mg/ml, preferably about 100 mg/ml at a temperature between about 25°C and about 60°C, preferably about 40°C. Particulate matter remaining in the solution may be removed by conventional means such as filtration.

Thereafter, the compound solution, maintained at a temperature of about 40°C, is mixed 1:1 (V/V) with an aqueous acid solution (also at about 40°C) having an acid concentration ranging between about 0.05 N and about 2 N, preferably about 1.7 N. The resulting mixture is further incubated at 40°C for a period of time effective for microsphere formation, as observed by light microscopy. In practicing this invention, the preferred order of addition is to add the compound solution to the aqueous acid solution.

Suitable acids for microsphere formation include any acid which does not
(a) adversely effect the modified amino acids, poly amino acids, or peptides *e.g.*, initiate or propagate chemical decomposition;
(b) interfere with microsphere formation;
(c) interfere with microsphere incorporation of the active agent cargo; and
(d) adversely interact with the active agent cargo.

Preferred acids for use in this aspect include acetic acid, citric acid, hydrochloric acid, phosphoric acid, malic acid and maleic acid.

A microsphere stabilizing additive may be incorporated into the aqueous acid solution or into the compound or cargo solution prior to the microsphere formation process. With some active agents the presence of such additives promotes the stability and/or dispersibility of the microspheres in solution.

The stabilizing additives may be employed at a concentration ranging between about 0.1 and 5 % (w/v), preferably about 0.5 % (w/v). Suitable, but non-limiting, examples of microsphere stabilizing additives include gum acacia, gelatin, methyl cellulose, polyethylene glycol, polypropylene glycol, carboxylic acids and salts thereof, and polylysine. The preferred stabilizing additives are gum acacia, gelatin and methyl cellulose.

Under the above conditions, the compound molecules form hollow or solid matrix type microspheres wherein the cargo is distributed in a carrier matrix or capsule type microspheres encapsulating liquid or solid cargo. If the compound microspheres are formed in the presence of a soluble material, *e.g.*, a pharmaceutical agent in the aforementioned aqueous acid solution, this material will be encapsulated within the microspheres. In this way, one can encapsulate pharmacologically active materials such as peptides, proteins, and polysaccharides as well as charged organic molecules, *e.g.*, antimicrobial agents, which normally have poor bioavailability by the oral route. The amount of pharmaceutical agent which may be incorporated by the microsphere is dependent on a number of factors which include the concentration of agent in the solution, as well as the affinity of the cargo for the carrier. The compound microspheres do not alter the physiological and biological properties of the active agent. Furthermore, the encapsulation process does not alter the pharmacological properties of the active agent. Any pharmacological agent can be incorporated within the microspheres. The system is particularly advantageous for delivering chemical or biological agents which otherwise would be destroyed or rendered less effective by conditions encountered within the body of the animal to which -it is administered, before the microsphere reaches its target zone (*i.e.,* the area in which the contents of the microsphere are to be released) and for delivering pharmacological agents which are poorly absorbed in the gastro-intestinal tract. The target zones can vary depending upon the drug employed.

The particle size of the microsphere plays an important role in determining release of the active-agent in the targeted area of the gastro-intestinal tract. The preferred microspheres have diameters between about ≤ 0.1 microns and about 10 microns, preferably between about 0.5 microns and about 5 microns. The microspheres are sufficiently small to release effectively the active agent at the targeted area within the gastro-intestinal tract such as, for example, between the stomach and the jejunum. Small microspheres can also be administered parenterally by being suspended in an appropriate carrier fluid (*e.g.,* isotonic saline) and injected directly into the circulatory system, intramuscularly or subcutaneously. The mode of administration selected will vary, of course, depending upon the requirement of the active agent being administered. Large amino acid microspheres (>50 microns) tend to be less effective as oral delivery systems.

The size of the microspheres formed by contacting compounds with water or an aqueous solution containing active agents can be controlled by manipulating a variety of physical or chemical parameters, such as the pH, osmolarity or ionic strength of the encapsulating solution, size of the ions in solution and by the choice of acid used in the encapsulating process.

The administration mixtures are prepared by mixing an aqueous solution of the carrier with an aqueous solution of the active ingredient, just prior to administration. Alternatively, the carrier and the biologically or chemically active ingredient can be admixed during the manufacturing process. The solutions may optionally contain additives such as phosphate buffer salts, citric acid, acetic acid, gelatin, and gum acacia.

Stabilizing additives may be incorporated into the carrier solution. With some drugs, the presence of such additives promotes the stability and dispersibility of the agent in solution.

The stabilizing additives may be employed at a concentration ranging between about 0.1 and 5 % (W/V), preferably about 0.5 % (W/V). Suitable, but non-limiting, examples of stabilizing additives include gum acacia, gelatin, methyl cellulose, polyethylene glycol, carboxylic acids and salts thereof, and polylysine. The preferred- stabilizing additives are gum acacia, gelatin and methyl cellulose.

The amount of active agent is an amount effective to accomplish the purpose of the particular active agent. The amount in the composition typically is a pharmacologically or biologically effective amount. However, the amount can be less than a pharmacologically or biologically effective amount when the composition is used in a dosage unit form, such as a capsule, a tablet or a liquid, because the dosage unit form may contain a multiplicity of carrier/biologically or chemically active agent compositions or may contain a divided pharmacologically or biologically effective amount. The total effective amounts can then be administered in cumulative units containing, in total, pharmacologically or biologically or chemically active amounts of biologically or pharmacologically active agent.

The total amount of active agent, and particularly biologically or chemically active agent, to be used can be determined by those skilled in the art. However, it has surprisingly been found that with some biologically or chemically active agents, the use of the presently disclosed carriers provides extremely efficient delivery, particularly in oral, intranasal, sublingual, intraduodenal, or subcutaneous systems. Therefore, lower amounts of biologically or chemically active agent than those used in prior dosage unit forms or delivery systems can be administered to the subject, while still achieving the same blood levels and therapeutic effects.

The amount of carrier in the present composition is a delivery effective amount and can be determined for any particular carrier or biologically or chemically active agent by methods known to those skilled in the art.

Dosage unit forms can also include any of excipients; diluents; disintegrants; lubricants; plasticizers; colorants; and dosing vehicles, including, but not limited to water, 1,2-propane diol, ethanol, olive oil, or any combination thereof.

Administration of the present compositions or dosage unit forms preferably is oral or by intraduodenal injection.

The delivery compositions of the present invention may also include one or more enzyme inhibitors. Such enzyme inhibitors include, but are not limited to, compounds such as actinonin or epiactinonin and derivatives thereof. These compounds have the formulas below: 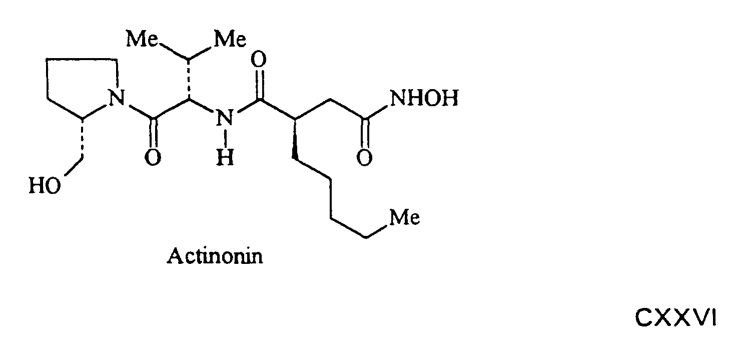 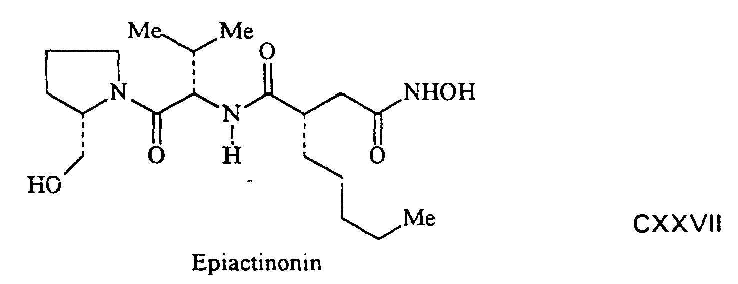 Derivatives of these compounds are disclosed in U.S. Patent No. 5,206,384. Actinonin derivatives have the formula: 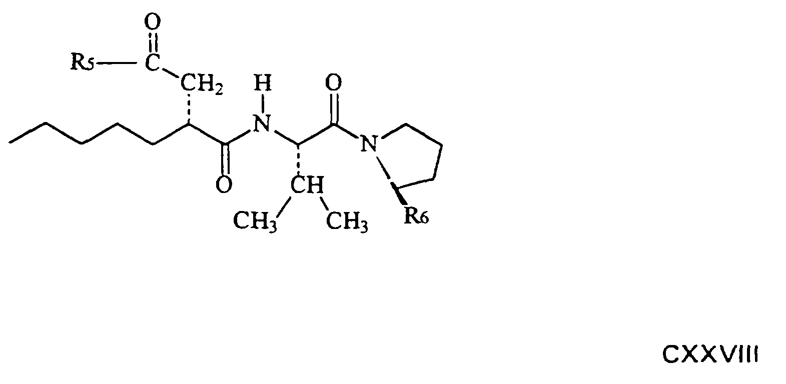 wherein R⁵ is sulfoxymethyl or carboxyl or a substituted carboxy group selected from carboxamide, hydroxyaminocarbonyl and alkoxycarbonyl groups; and R⁶ is hydroxyl, alkoxy, hydroxyamino or sulfoxyamino group. Other enzyme inhibitors include, but are not limited to, aprotinin (Trasylol) and Bowman-Birk inhibitor.

The compounds and compositions of the subject invention are useful for administering biologically or chemically active agents to any animals such as birds; mammals, such as primates and particularly humans; and insects. The system is particularly advantageous for delivering chemically or biologically or chemically active agents which would otherwise be destroyed or rendered less effective by conditions encountered before the active agent its target zone (i.e. the area in which the active agent of the delivery composition are to be released) and within the body of the animal to which they are administered. Particularly, the compounds and compositions of the present invention are useful in orally administering active agents, especially those which are not ordinarily orally deliverable.

### DESCRIPTION OF THE PREFERRED EMBODIMENTS

The following examples illustrate the invention without limitation. All parts are given by weight unless otherwise indicated.

### Example 1

Compound XIX was prepared as follows:

A 3 L three-neck round bottom flask was fitted with an overhead mechanical stirrer and a thermometer, and the flask was cooled in an icebath. A solution of 8-aminocaprylic acid (100.0 g, 0.65 moles) in 2 M aqueous sodium hydroxide (1.4 L) was charged into the round bottom flask. The temperature of the solution was kept at about 5°C, and *O-*acetylsalicyloyl chloride (198.6 g, 0.76 moles, 1.2 equiv.) was added portionwise over 7 hours. The mixture was stirred at 5°C for 12 hours to yield a yellow homogenous solution. The solution was acidified with 1 M hydrochloric acid to pH 6.8 and was extracted with ethyl acetate (2 x 600 mL). The pH of the aqueous layer was readjusted to 6.3 and was further extracted with ethyl acetate (2 x 600 mL). The organic layers were combined, dried over anhydrous sodium sulfate, filtered, and evaporated under reduced pressure. The residue was redissolved in a minimum volume of 2 M aqueous sodium hydroxide, and the pH of the solution was between 9.5 and 10. The mixture was acidified with stirring with 1 M hydrochloric acid to pH of about 6.2, and a solid was formed. The solid was filtered, washed with water (3 x 300 mL), and recrystallized from 55% methanol/water (v/v) to yield Compound XVIII as an off-white solid (99.7 g, 57%).

Properties are listed below.
mp 116-117°C. ¹H NMR (300 MHz, DMSO-d₆) δ: 12.70 (1 H, br s), 11.95 (1 H, br s) 8.81 (1H, t), 7.82 (1H. m), 7.38 (1H, m), 6.84 (2H, m), 2.36 (2H, q), 2.18 (2H, t), 1.50 (4H, br m), 1.28 (6H, m), Anal. Calcd for C₁₅H₂₁NO₄: C, 64.50; H, 7.58;1 N, 5.02. Found: C, 64.26; H, 7.81; N, 4.93.

Similar procedures were used to prepare Compounds IV, XXVII, XXVIII.

Properties are listed below.
- Compound IV:: Anal. Calcd for C₁₁H₁₃NO₄: C, 59.9, H, 5.87, N, 6.27 Found: C, 58.89, H, 5.85, N, 6.07. ¹H NMR (300MHz, DMSO-d₆): δ 1.8 (2H, m) 2.3 (2H, t) 3.1 (2H,q) 6.9 (2H, t) 7.4 (1H, t) 7.8 (1 H, d) 8.85 (1 H, t) 12.0 (1 H, s) 12.15 (1 H, s)
- Compound XXVII:: Anal. Calcd for C₁₈H₂₇NO₄: C, 67:25, H, 8.48, N, 4.36 Found: C, 67.23, H, 8.57, N, 4.20. ¹H NMR (300MHz, DMSO-d₆): δ 1.22-1.26 (m, 12H) 1.45-1.51 (m, 4H) 2.16 (t, 2H) 3.25 (qt, 2H), 6.85 (t, 2H), 7.37 (t, 1 H), 7.81 (d, 1H), 8.79 (t, 1H), 11.95 (s, 1 H), 12.72 (s, 1 H)
- Compound XXVIII:: ¹H NMR (300MHz, DMSO-d₆): δ 1.26 (8H, br m), 1.49 (4H, m), 2.17 (2H, t), 3.26 (2H, m), 6.86 (2H, m), 7.37 (1H. m), 7.83 (1H, m), 8.80 (1H, t), 11.95 (1H, s), 12.73 (1H, s).

### Example 1A

An alternate synthesis of compound XIX was as follows:

A 5 L three-neck round bottom flask was fitted with a heating mantle, an overhead mechanical stirrer, an addition funnel, and a thermometer. The reaction was performed under an argon atmosphere. Hydroxylamine-*O*-sulfonic acid (196.7 g, 1.74 moles, 1.10 equiv.) and formic acid (1 L) were charged into the round bottom flask and stirred to form a white slurry. A solution of cyclooctanone (200.0 g 1.58 moles, 1.0 equiv.) in formic acid (600 mL) was added dropwise to the white slurry via the addition funnel. After the addition, the addition funnel was repiaced by a reflux condenser, and the reaction was heated to reflux (internal temperature about 105°C) for 1 hour to give a brown solution. After the solution was cooled to room temperature, it was poured into a mixture of saturated aqueous ammonium chloride (1.5 L) and water (1.5 L). The aqueous mixture was extracted with chloroform (3 x 1200 mL). The combined chloroform layers were transferred into a beaker, and saturated sodium bicarbonate (2 L) was added slowly. The chloroform layer was then separated, dried over anhydrous sodium sulfate, and evaporated under reduced pressure to afford a brown oil. The oil was placed in a 500 mL round bottom flask with a magnetic stirrer. The round bottom flask was placed in a silicon oil bath and was fitted with a short path vacuum distillation head equipped with a thermometer. A Cow-type receiver was connected to three 250 mL flasks. 2-Azacyclononanone (145 g, 65%, mp 64-69°C) was obtained by vacuum distillation (fraction with head temperature range from 80 to 120°C at pressures between 3.0 and 3.4 mmHg).

A 5 L three-neck round bottom flask was fitted with a heating mantle, an overhead mechanical stirrer, a reflux condenser, and a 29 thermometer. A suspension of 2-azacyclononanone (83 g, 0.59 moles, 1.0 equiv.) in 5 M aqueous sodium hydroxide (650 mL. 8:23 moles, 5.5 equiv.) was charged into the round bottom flask. The mixture was heated to reflux (internal temperature about 110° C) for 4 hours to yield a clear yellow solution. The heating mantle and reflux condenser were removed. After the solution cooled to room temperature, it was diluted with water (650 mL) and cooled further in an ice bath. Finely ground *O*-acetylsalicyloyl chloride (114.7 g, 0.59 moles, 1.0 equiv.) was added portionwise to the solution with stirring and continued cooling over 1 hour. After an additional 30 minutes, the icebath was removed and stirring was continued at ambient temperature for 21 hours to give a brownish yellow solution. The stirred mixture was acidified with 2 M sulfuric acid (about 850 mL) to a pH of about 1, and a yellow solid was formed. The solid was collected by filtration and was dissolved in warm methanol (1.7 L). Activated charcoal (about 5 g) was added to the methanol, and the solution was stirred for 10 minutes. The activated charcoal was removed by filtration, and the charcoal residue was washed with additional 300 mL methanol. Water (2 L) was added to the combined filtrates (*i.e.* the 2 L methanol), and an off-white solid precipitated upon standing at 4°C overnight. The crude product was filtered and was recrystallized from 65% methanol/water (v/v) to yield Compound XIX (69.1 g, 42%) as off-white solid.

Properties are listed below:
mp 116-117°C; HPLC, ¹H NMR and Anal. Calcd for C₁₅H₂₁NO₂: C, 64.50; H, 7.58; N, 5.02. Found: C. 64.26; H, 7.81; N, 4.93.

### Example 2

Compound XXXI was prepared as follows:

**1-Aminoundec-10-ene.** A mixture of 10-undecene-1-ol (5.00g, 29.36 mmol, 1 equiv), triphenylphosphine (7.70g, 29.36 mmol, 1 equiv) and phthalimide (4.32g, 29.36 mmol, 1 equiv) in dry tetrahydrofuran (THF, 30 mL) was stirred vigorously under argon. Diethyl azodicarboxylate (DEAD, 5.11g, 29.36 mmol, 1 equiv) was diluted with THF (12 mL) and added dropwise by syringe. After the addition, the reaction was stirred at room temperature for 4 hours. The solvent was evaporated under vacuum and ether (30 mL) was added to precipitate the triphenylphosphine oxide and hydrazine dicarboxylate which were removed by filtration. The precipitate was rinsed with ether (2 x 30 mL) and the combined filtrates were evaporated to afford a yellow solid. The yellow solid was triturated with warm hexanes (3 x 50 mL) and filtered. The combined hexanes were evaporated to give 1-phthalimidylundec-10-ene as a yellow wax.

The yellow wax was dissolved in an ethanolic solution (38 mL) of hydrazine hydrate (1.47g, 1 equiv, 29.36 mmol). The mixture was heated at reflux for 2 hours. After the mixture was cooled to room temperature, concentrated hydrochloric acid (30 mL) was added and the solid was filtered through a sintered glass filter. The residue was washed with water (50 mL) and the combined filtrates were evaporated to provide a yellow solid. The yellow solid was redissolved in 1 M NaOH (100 mL) and extracted with ether (2 x 50 mL). The ether was dried and evaporated to provide a yellow oil. The oil was purified by Kugelrohr distillation (ca: 0.1 mmHg, 100 °C) to provide 1-aminoundec-10-ene (2) as a light yellow oil (3.29 g, 66%).

Properties are listed below.
¹H NMR (300 mHz, DMSO-d₆); δ 1.23 (14H, br m), 1.99 (2H, m), 2.48 (2H, m), 4.94 (2H, m), 5.77 (1H, m).

**1-(*O*-Acetylsalicyloylamino)undec-10-ene.** *O*-Acetylsalicyloyl chloride (3.82 g, 19.25 mmol, 1 equiv) in THF (30 mL) was cooled in an ice bath. Triethylamine (1.95 g, 19.25 mmol, 1 equiv), followed by 1-aminoundec-10-ene (3.26 g, 19.25 mmol, 1 equiv) in THF (10mL) were added via syringe. The ice bath was removed and the reaction was stirred at room temperature for 3.5 hours. After removal of the solvent, the residue was dissolved in EtOAc (50 mL) and washed with water (2 x 30 mL). The organic layer was dried and evaporated to afford 1-(*O*-acetylsalicyloyl-amino)undec-10-ene as a colorless oil, in a quantitative yield, 6.59 g.

Properties are listed below.
¹H NMR (300 mHz, DMSO-d₆: δ1.26 (12H, br s), 1.47 (2H,m), 1.99(2H,m), 2.19 (3H,s), 3.15 (2H, q), 4.95 (2H, m), 5.78 (1H, m), 7.15 (1H, m), 7.30 (1H, m), 7.50 (2H, m) 8.24 (1H. t).

### COMPOUND XXXI

1-(*O*-Acetylsalicyloylamino)under-10-ene (6.59 g, 19.25 mmol, 1 equiv) in dichloromethane (108 mL) was added to a mixture of water (108 mL), sulfuric acid (9M, 13 mL), glacial acetic acid (2.16 mL) and methyltrialkyl(C₈- C₁₀)ammonium chloride (0.32 g) (Adogen^{®} 464, available from Aldrich Chemical Co.). The mixture was stirred vigorously in an ice bath and potassium permanganate (9.13 g, 57.75 mmol, 3 equiv) was added in portions over 1.5 hours. After the addition, the ice bath was removed and the resultant purple solution was stirred at room temperature for 20 hours. The solution was cooled in an ice bath and sodium bisulfite (6.8 g) was added to dissipate the excess permanganate. The organic layer was separated and the aqueous layer was extracted with ethyl acetate (2 x 50 mL). The combined organic layers were washed with brine (50 mL), dried and evaporated. Sodium hydroxide (2M, 50 mL) was added to the residue and stirred for 30 min. The solution was diluted with water (50 mL), washed with ether (50 mL) and acidified to pH 1 with 2M hydrochloric acid. A solid formed and was collected by filtration. Recrystallization of the solid from 65 % MeOH/H₂O gave XXXI as a tan solid (2.78 g, 47% based on the amine).

Properties are listed below.
¹H NMR (300 mHz, DMSO-d₆): δ 1.24 (10H, br m), 1.51 (4H, m), 2.17 (2H, t), 3.27 (2H, m), 6.86 (2H, m), 7.37 (1H m), 7.82 (1H, m), 8.80 (1H. t), 11.95 (1H, s), 12.72 (1H, s).

The other compounds of the invention can be readily prepared by following the procedures described in Examples 1-2.

### Examples 5-15 - In Vivo Evaluation of Recombinant Growth Hormone in Rats

Dosing compositions were prepared by mixing the modified amino acids and recombinant human growth hormone (rhGH) as listed in Table 1 below in a phosphate buffer solution at a pH of about 7-8.

Rats were administered the dosing composition by sublingual, oral gavage, intraduodenal administration, or colonic administration. Delivery was evaluated by using an ELISA assay for rhGH from Medix Biotech, Inc. For intracolonic administration, a sample was prepared and dosed to fasted rats at 25 mg/kg of carrier in a buffered solution containing propylene glycol (0-50%) and 1 mg/kg rhGH.

Results are illustrated in Table 1 below.

### Comparative Example 5A

rhGH (6 mg/ml) was administered by oral gavage to a rat, and delivery was evaluated according to the procedure of Example 5.

Results are illustrated in Table 1 below.

| Table 1 | | | | | |
|---|---|---|---|---|---|
| *In Vivo* Delivery of rhGH | | | | | |
| Example | Carrier | Carrier Dose (mg/kg) | Drug Dose (mg/kg) | Method of Administration | Mean Peak Serum Levels of rhGH (ng/mL) |
| 5A | none | 0 | 6 | oral | <10+/-10 |
| 11 | XIX | 200 | 3 | oral | 60.92 + /-26.3 |
| 12 | XIX | 25 | 1 | colonic | 111.52+/-16.4 |
| 13 | XIX | 100 | 3 | sublingual | 119.14+/-65.6 |
| 14 | XIX | 25 | 1 | intranasal | 92.7+/-73.2 |
| 15 | XXVII | 25 | 1 | colonic | 73.72+/-4.9 |

### Examples 16-27 - In Vivo Evaluation of Recombinant Growth Hormone in Rats

### Preparation of Dosing solutions.

The delivery agents were reconstituted with distilled water and adjusted to pH 7.2-8.0 with either aqueous hydrochloric acid or aqueous sodium hydroxide. A stock solution of rhGH was prepared by mixing rhGH, D-mannitol and glycine and dissolving this mixture in 2% glycerol/water. The stock solution was then added to the delivery agent solution. Several delivery agent to active agent ratios were studied.

### In vivo experiments.

Male Sprague-Dawley rats weighing 200-250g were fasted for 24 hours and administered ketamine (44 mg/kg) and chlorpromazine (1.5 mg/kg) 15 minutes prior to dosing. The rats were administered one of the dosing solutions described above by subcutaneous injection, intranasal instillation, or sublingual instillation. Blood samples were collected serially from the tail artery for serum calcium concentration determination or serum rhGH concentrations. The dose of rhGH administered in these experiments was 0.1 mg/kg.

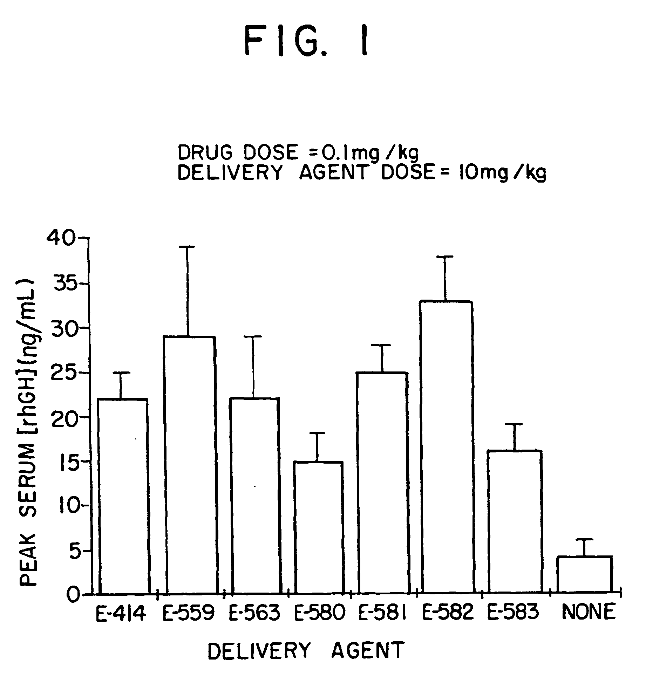

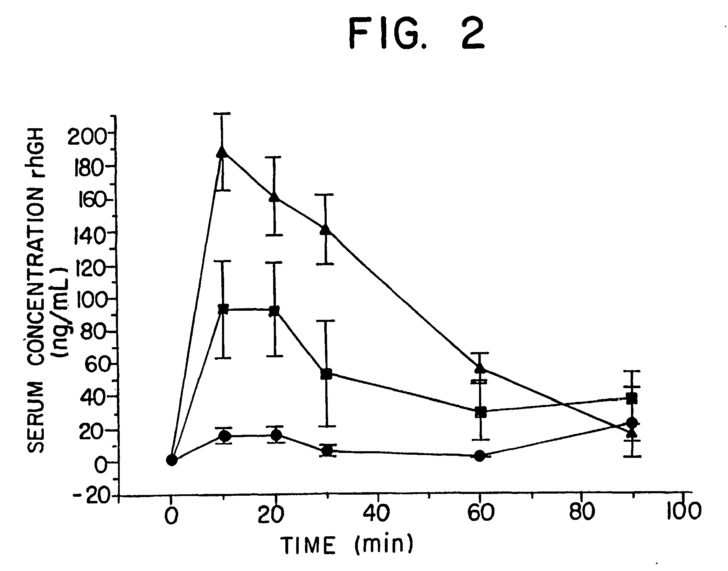

Serum rhGH concentrations were quantified by an rhGH enzyme immunoassay test kit. The results are given in Table 2 and Figures 1 and 2.

In Figure 2 the circles represent the response following SL dosing of an aqueous solution of compound XIX and rhGH. The squares represent the response following IN dosing of an aqueous solution of compound XIX and rhGH. The triangles represent the response following IC dosing of an aqueous solution of compound XIX and rhGH. The dose of compound XIX was 25 mg/kg and the dose of rhGH was 1 mg/kg.

### Comparative Example 16A

rhGH (1 mg/kg) was administered by oral gavage to a rat, and delivery was evaluated according to the procecure of Example 16.

Results are illustrated in Table 2 below.

| **Table 2** | | | |
|---|---|---|---|
| Delivery Agent Enhancement of Recombinant Human Growth Hormone (rhGH) Bioavailability Administered by Subcutaneous Administration. | | | |
| Example | Deliver Ageny | Delivery Agent Dose (mg/kg) | Peak Serum [rhGH] (ng/mL) |
| 16 | XIX | 1.0 | 22 ± 3 |
| 16A | None | 0.0 | 4 ± 2 |
| 17 | XIX | 2.5 | 25 ± 5 |
| 18 | XIX | 25 | 30 ± 6 |
| 27 | CIX | 1.0 | 16 ± 3 |

### Examples 28-33 - In Vivo Evaluation of Interferon in Rats

Dosing compositions were prepared by mixing the modified amino acid compounds and interferon α2b as listed in Table 3 below in a Trizma^{®} hydrochloride buffer solution (Tris-HCl) at a pH of about 7-8. Propylene glycol (0-25%) was added as a solubilizing agent, if necessary.

Rats were administered the dosing composition by oral gavage, intraduodenal administration, or intracolonic administration. Delivery was evaluated by use of an ELISA assay for human interferon α from Biosource, Inc.

Results of intracolonic administration are illustrated in Table 3 below.

### Comparative Example 28A

Interferon α2b (250 µg/kg) was administered intracolonically to rats, and delivery was evaluated according to the procedure of Example 14.

Results are illustrated in Table 3 below.

| Table 3 | | | | |
|---|---|---|---|---|
| *In Vivo* Delivery of Interferon by Intracolonic Administration | | | | |
| Example | Carrier | Carrier Dose (mg/kg) | Interferon Dose (*µ*g/kg) | Mean Peak Serum Levels of Interferon (pg/mL) |
| 28A | none | 0 | 250 | 0 |
| 31 | XIX | 100 | 250 | 11193 + /-8559 |

### Examples 34-37 - In Vivo Evaluation of Salmon Calcitonin in Rats

Dosing compositions were prepared by mixing the modified amino acids and salmon calcitonin as listed in Table 4 below. 400 mg of carrier were added to 2.9 mL of 25% aqueous propylene glycol. The resultant solution was stirred, and the pH was adjusted to 7.2 with sodium hydroxide (1.0 N). Water was added to bring the total volume to 2.0 mL. The sample had a final carrier concentration of 200 mg/mL. Calcitonin (10 µg) was added to the solution. The total calcitonin concentration was 2.5 µg/mL.

For each sample a group of fasted rats were anesthetized. The rats were administered the dosing composition by oral gavage, intracolonic instillation, or intraduodenal administration. Blood samples were collected serially from the tail artery. Serum calcium was determined by testing with a Calcium Kit (Sigma Chemical Company, St. Louis, Missouri, USA).

Results are illustrated in Table 4 below.

| **Table 4** | | | | | |
|---|---|---|---|---|---|
| *In Vivo* Delivery of Calcitonin | | | | | |
| Example | Carrier | Carrier Dose (mg/kg) | Drug Dose (*µ*g/kg) | Method of Administration | Maximum Decrease in Serum Calcium (% below baseline) |
| 36 | XIX | 10 | 3 | intracolonic | 26.49+/-12.3 |
| 37 | XIX | 200 | 7.5 | oral | 25.48+/-4.7 |

### Examples 38-43 - In Vivo Evaluation of Salmon Calcitonin in Rats

### Preparation of Dosing solution.

The delivery agents were reconstituted with distilled water and adjusted to pH 7.2-8.0 with either aqueous hydrochloric acid or aqueous sodium hydroxide. A stock solution of sCT was prepared by dissolving sCT in citric acid (0.085N). The stock solution was then added to the delivery agent solution. Several different delivery agent to active agent ratios were studied.

### In vivo experiments.

Male Sprague-Dawley rats weighing 200-250g were fasted for 24 hours and administered ketamine (44 mg/kg) and chlorpromazine (1.5 mg/kg) 15 minutes prior to dosing. The rats were administered one of the dosing solutions described above by subcutaneous injection. Blood samples were collected serially from the tail artery for serum calcium concentration.

Serum calcium concentrations were quantified by the o-cresolphthalein complexone method (Sigma) using a UV/VIS spectrophotometer (Perkin Elmer). The results are given in Table 5.

### Examples 38A

Salmon calcitonin was administered by ora! gavage to rats, and delivery was evaluated according to the Procedure of Example 38. The results are given in Table 5 below.

| Table 5 | | | |
|---|---|---|---|
| Delivery Agent Enhancement of Salmon Calcitonin (sCT, dosed at 0.2 µg/kg) Bioavailability Administered by Subcutaneous Administration. | | | |
| Example | Deliver Ageny | Delivery Agent Dose (µg/kg) | Percent Decrease in Serum Calcium |
| 38 | XIX | 2 | 17 ± 3 |
| 38A | None | 0 | 17 ± 2 |
| 39 | XIX | 20 | 25 ± 4 |
| 40 | XIX | 200 | 25 ± 5 |
| 41 | XIX | 2000 | 26 ± 5 |

### Examples 44-50 - In Vivo Evaluation of Heparin in Rats

Dosing compositions were prepared by mixing the modified amino acids and heparin as listed in Table 4. In a test tube, 900 mg of carrier were dissolved in 3 mL of propylene glycol, and 0.299g of sodium heparin was dissolved in 3 mL of water. The solutions were mixed by vortex. Sodium hydroxide (10M) was added to the resulting mixture until a solution was obtained. The pH was then adjusted to 7.4 + /-0.5 with concentrated hydrochloric acid, and the final solution was sonicated at 40°C for 30 minutes.

A group of fasted, conscious rats were administered the dosing compositions by oral gavage. Blood samples were collected by cardiac puncture following the administration of ketamine (44 mg/kg)., Heparin activity was determined by utilizing the activated partial thromboplastim time (APTT) according to the method of Henry, J.B., *Clinical Diagnosis and Management by Laboratory Methods;* Philadelphia, PA; WB Saunders (1979).

Results are illustrated in Table 6 below.

### Comparative Example 44A

Heparin (100 mg/kg) was administered by oral gavage to rats, and heparin activity was determined according to the procedure of Example 44.

Results are illustrated in Table 6 below.

| **Table 6** | | | | |
|---|---|---|---|---|
| *In Vivo* Delivery of Heparin by Oral Administration | | | | |
| Example | Carrier | Carrier Dose (mg/kg) | Drug Dose (mg/kg) | Mean Peak APTT (sec) |
| 44A | none | none | 100 | 20.7 + /-0.17 |
| 48 | XIX | 300 | 100 | 119.99 + /-56.3 |
| 49 | XXXI | 50 | 25 | 127.56 + /- 22.97 |
| 50 | XXXI | 50 | 10 | 50.85 +/-9.1 |

### Example 51

The method of Example 44 was followed, substituting low molecular weight heparin for the heparin and varying the amounts of propylene glycol and water for solubilization as, necessary.

### Examples 50-58 - In vivo Evaluation of Parathyroid Hormone in Rats

### Preparation of dosing solutions.

The delivery agents were reconstituted with distilled water and/or propylene glycol and adjusted to an apparent pH of 7.2-8.0 with either aqueous hydrochloric acid or aqueous sodium hydroxide. A stock solution of parathyroid hormone was prepared by dissolving parathyroid hormone in water. The parathyroid hormone solution was then added to the delivery agent solution. Several different delivery agent to active agent ratios were studied.

### In vivo experiments.

Male Sprague-Dawley rats weighing 200-250g were fasted for 24 hours and administered ketamine (44 mg/kg) and chlorpromazine (1.5 mg/kg) 15 minutes prior to dosing. The rats were administered one of the dosing solutions described above by oral gavage or intracolonic instillation. Blood samples were collected serially from the tail artery for serum determination of parathyroid hormone concentration. Serum parathyroid hormone concentrations were quantified by a parathyroid hormone radioimmunoassay test kit.

### In vivo Oral administration.

Oral administration of solutions containing parathyroid hormone (PTH) and the non-α-amino acid delivery agents was tested *in vivo* in rats. The result show a significant increase in the oral bioavailability of parathyroid hormone as compared to similar administration of the active agent alone. Data are presented in Table 7.

| Table 7 | | | | | |
|---|---|---|---|---|---|
| Delivery Agent Enhancement of Parathyroid Hormone (PTH) Oral Bioavailability. | | | | | |
| Example | Carrier | Carrier Dose mg/kg | Method of Administration | Active Agent Dose (µg/kg) | Peak Serum [PTH] (pg/mL) |
| 51 | XIX | 100 | intracolonic | 25 | 130 ± 20 |
| 52 | XIX | 250 | oral | 100 | 75 ± 25 |
| 53 | XIX | 250 | oral | 25 | 20 ± 6 |

## Claims

1. A compound selected from the group consisting of 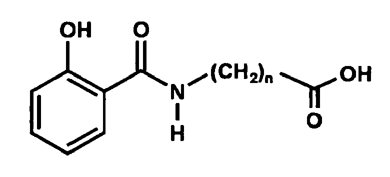 wherein
| Compound | n |
|---|---|
| XIX | 7 |
| XXVII | 10 |
| XXVIII | 8 |
| XXIX | 6 |
| XXX | 11 |
| XXXI | 9 |

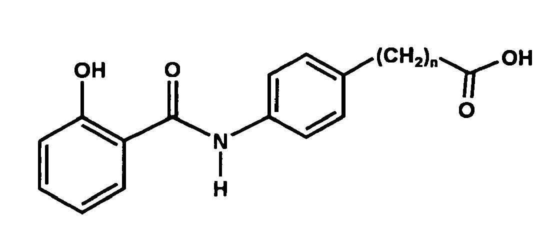
wherein
| Compound | n |
|---|---|
| LIV | 2 |

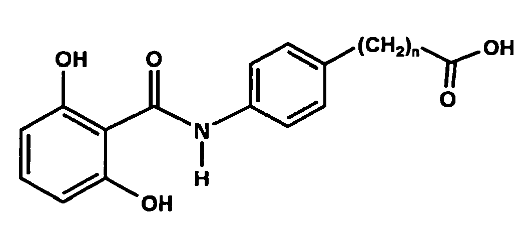
wherein
| Compound | n |
|---|---|
| LIII | 3 |
| LVI | 2 |

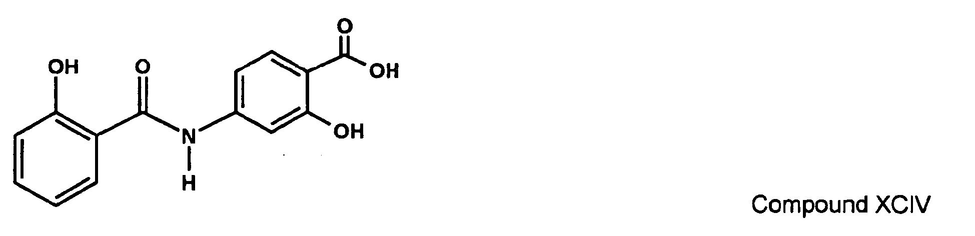

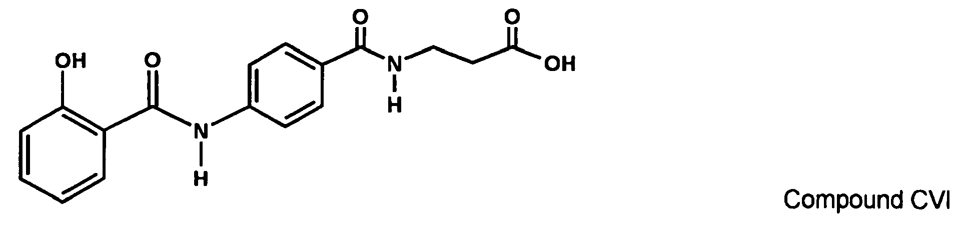

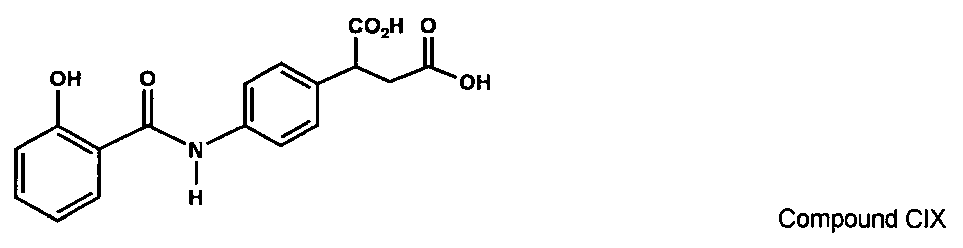
or salts thereof.

2. A composition comprising
a) an active agent;
b) a compound selected from the group consisting of 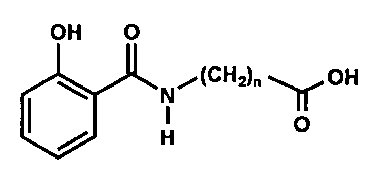
wherein
| Compound | n |
|---|---|
| IV | 3 |
| XIX | 7 |
| XXVII | 10 |
| XXVIII | 8 |
| XXIX | 6 |
| XXX | 11 |
| XXXI | 9 |

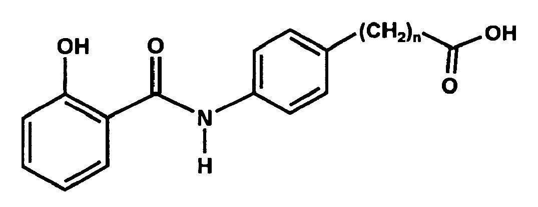
wherein
| Compound | n |
|---|---|
| LII | 1 |
| LIV | 2 |

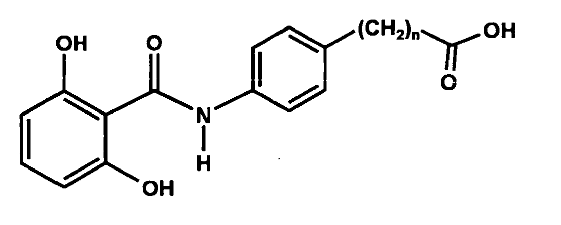
wherein
| Compound | n |
|---|---|
| LIII | 3 |
| LVI | 2 |

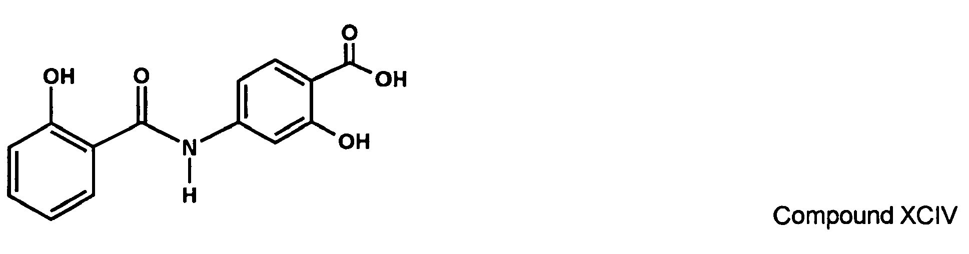

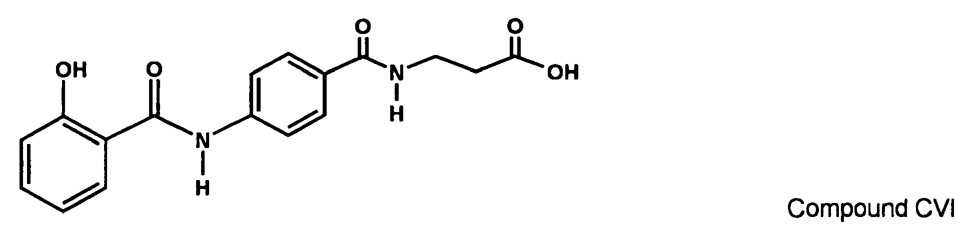

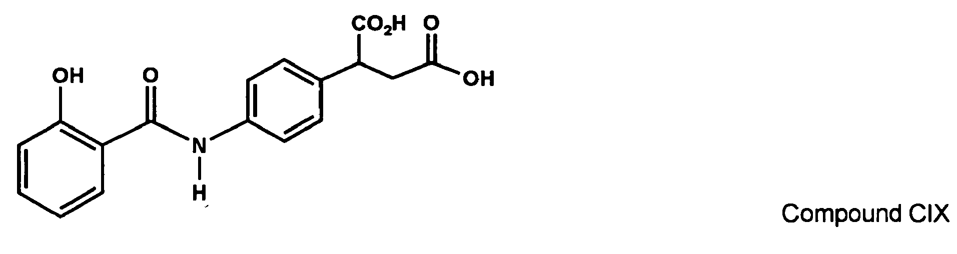
or salts thereof.

3. The composition according to claim 2, wherein said active agent is selected from the group consisting of a biologically active agent and a chemically active agent.

4. The composition according to claim 3, wherein said biologically active agent is selected from the group consisting of a peptide, a mucopolysaccharide, a carbohydrate, a lipid, a pesticide, or any combination thereof.

5. The composition according to claim 4, wherein said biologically active agent is selected from the group consisting of human growth hormone, bovine growth hormone, growth hormone-releasing hormone, an interferon, interleukin-II, insulin, heparin, calcitonin, erythropoietin, atrial naturetic factor, an antigen, a monoclonal antibody, somatostatin, adrenocorticotropin, gonadotropin releasing hormone, oxytocin, vasopressin, cromolyn sodium, vancomycin, parathyroid hormone, desferrioxamine (DFO), or any combination thereof.

6. The composition according to claim 2, wherein said composition is suitable for being administered orally, intranasally, sublingually, intraduodenally, intramuscularly or subcutaneously to an animal in need of an active agent.

7. The composition according to claim 6, wherein the active agent is a biologically active agent and the composition is suitable for being administered orally to an animal.

8. A dosage unit form comprising
(A) a composition as defined in claim 2; and
(B)
(a) an excipient,
(b) a diluent,
(c) a disintegrant,
(d) a lubricant,
(e) a plasticizer,
(f) a colorant,
(g) a dosing vehicle, or
(h) any combination thereof.

9. A dosage unit form according to claim 8, comprising a tablet, a capsule, or a liquid.

10. A method for preparing a composition, said method comprising mixing
(A) at least one biologically-active agent;
(B) at least one compound as defined in claim 1; and
(C) optionally a dosing vehicle.

11. A compound according to claim 1 of the following formula 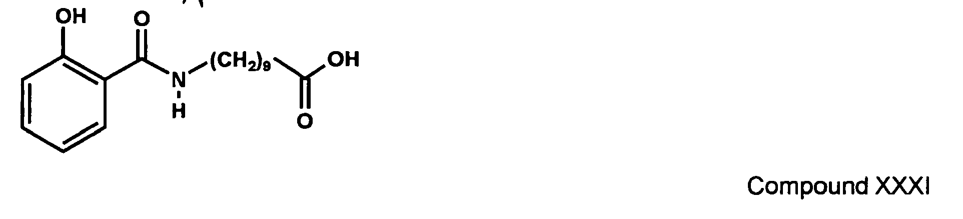 or a salt thereof.

12. A composition according to claim 3 comprising
(a) compound of the following formula 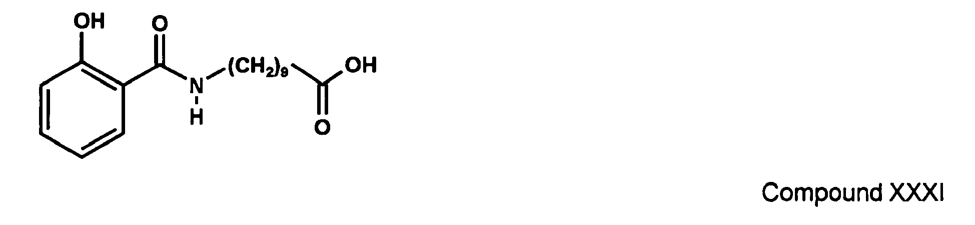 or a salt thereof; and
(b) a biologically active agent.

13. The composition of claim 12, wherein the biologically active agent is heparin.

14. A compound according to claim 1 of the following formula 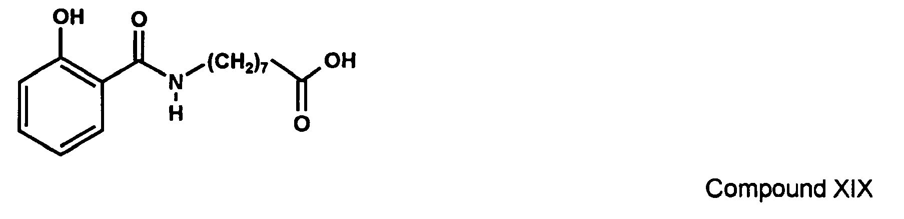 or a salt thereof.

15. A composition according to claim 3 comprising
(a) a compound of the following formula 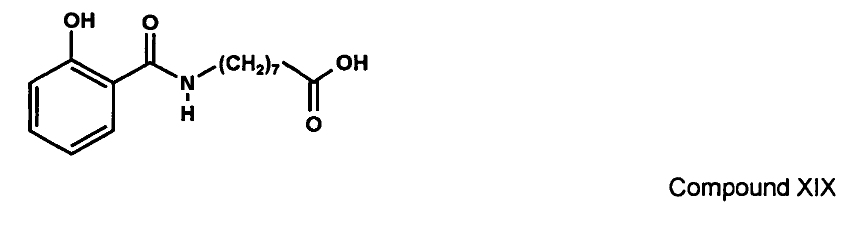 or a salt thereof; and
(b) a biologically active agent.

16. The composition of claim 15, wherein the biologically active agent is human growth hormone.

17. The composition of claim 15, wherein the biologically active agent is interferon.

18. The composition of claim 15, wherein the biologically active agent is calcitonin.

19. The composition of claim 18, wherein the biologically active agent is salmon calcitonin.

20. The composition of claim 15, wherein the biologically active agent is heparin.

21. The composition according to any of claims 12,13,15 to 20, wherein said composition is suitable for being administered orally.

22. A composition according to claim 3 comprising a compound of the following formula 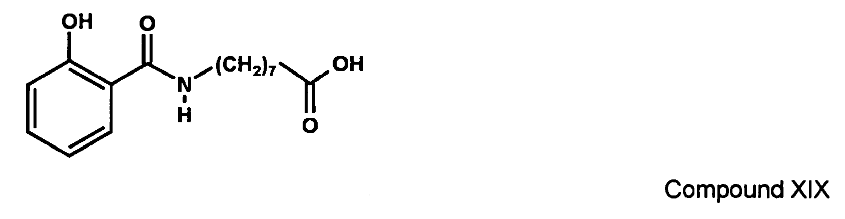 or a salt thereof; and
and parathyroid hormone.

## Patentansprüche

1. Eine Verbindung, ausgewählt aus der Gruppe, bestehend aus 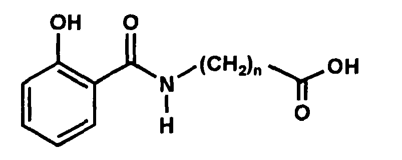 worin
| Verbindung | n |
|---|---|
| XIX | 7 |
| XXVII | 10 |
| XXVIII | 8 |
| XXIX | 6 |
| XXX | 11 |
| XXXI | 9 |

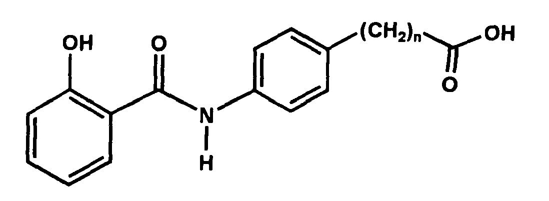
worin
| Verbindung | n |
|---|---|
| LIV | 2 |

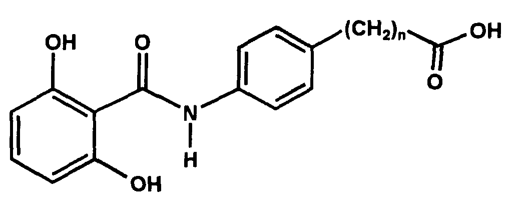
worin
| Verbindung | n |
|---|---|
| LIII | 3 |
| LVI | 2 |

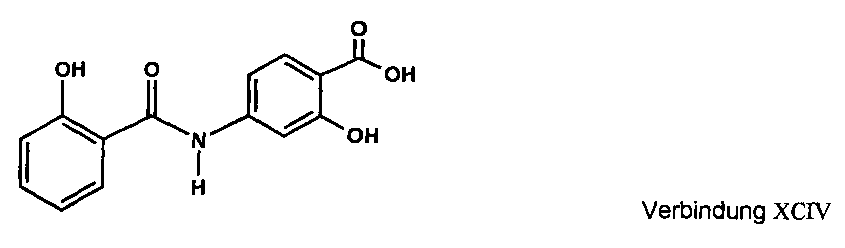

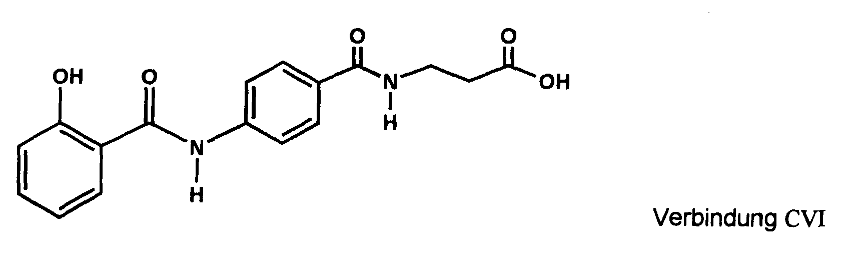

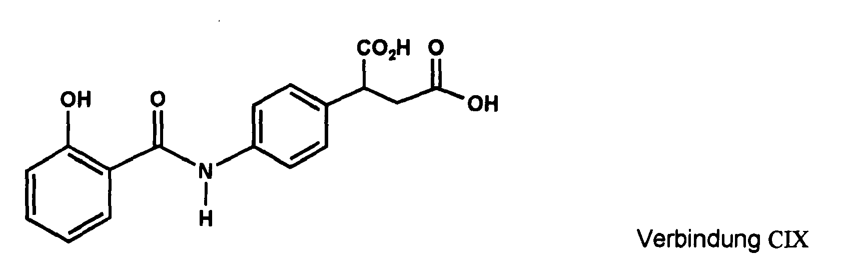
oder Salze derselben.

2. Eine Zusammensetzung, umfassend
a) einen Wirkstoff;
b) eine Verbindung, ausgewählt aus der Gruppe, bestehend aus 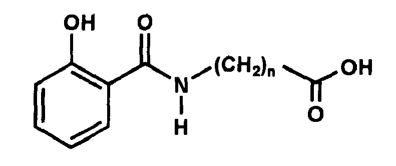
worin
| Verbindung | n |
|---|---|
| IV | 3 |
| XIX | 7 |
| XXVII | 10 |
| XXVIII | 8 |
| XXIX | 6 |
| XXX | 11 |
| XXXI | 9 |

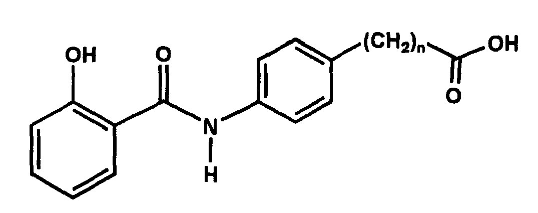
worin
| Verbindung | n |
|---|---|
| LII | 1 |
| LIV | 2 |

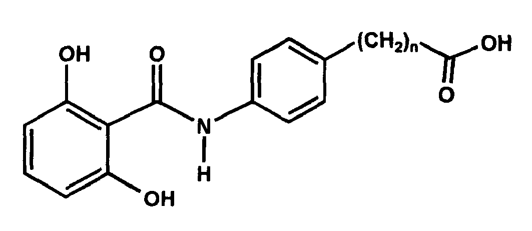
worin
| Verbindung | n |
|---|---|
| LIII | 3 |
| LVI | 2 |

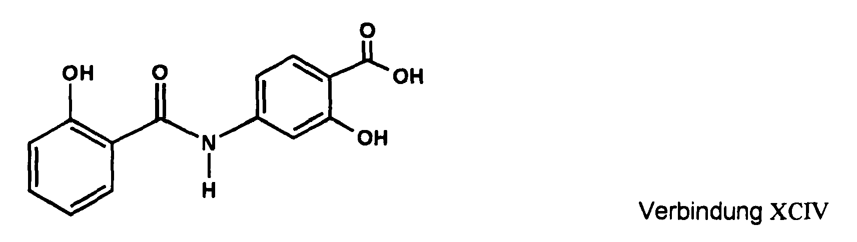

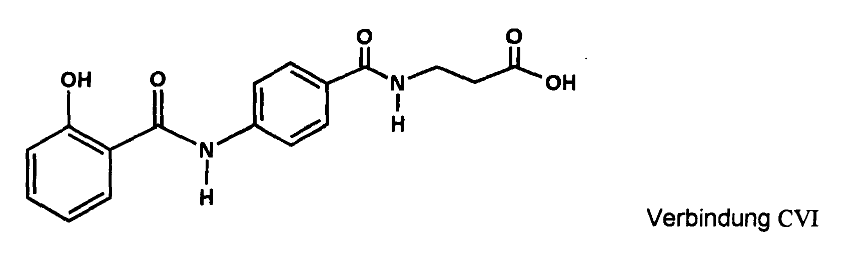

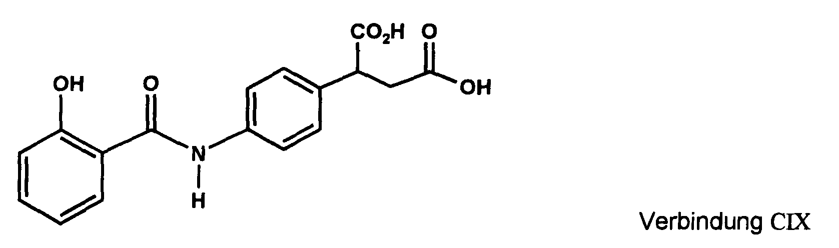
oder Salze derselben.

3. Die Zusammensetzung gemäß Anspruch 2, worin der Wirkstoff ausgewählt ist aus der Gruppe, bestehend aus einem biologisch aktiven Wirkstoff und einem chemisch aktiven Wirkstoff.

4. Die Zusammensetzung gemäß Anspruch 3, worin der biologisch aktive Wirkstoff ausgewählt ist aus der Gruppe, bestehend aus einem Peptid, einem Mucopolysaccharid, einem Kohlenhydrat, einem Lipid, einem Pestizid oder einer Kombination derselben.

5. Die Zusammensetzung gemäß Anspruch 4, worin der biologisch aktive Wirkstoff ausgewählt ist aus der Gruppe, bestehend aus einem menschlichen Wachstumshormon, Rinderwachstumshormon, Wachstumshormon-freisetzendem Hormon, einem Interferon, Interleukin-II, Insulin, Heparin, Calcitonin, Erythropoietin, atrialem natriuretischem Faktor, einem Antigen, einem monoklonalen Antikörper, Somatostatin, Adrenocorticotropin, Gonadotropin-freisetzendem Hormon, Oxytocin, Vasopressin, Cromolyn-Natrium, Vancomycin, Parathyroidhormon, Desferrioxamin (DFO) oder jeder Kombination derselben.

6. Die Zusammensetzung gemäß Anspruch 2, worin die Zusammensetzung geeignet ist, oral, intranasal, sublingual, intraduodenal, intramuskulär oder subkutan an ein Lebewesen verabreicht zu werden, welches einen Bedarf nach dem Wirkstoff hat.

7. Die Zusammensetzung gemäß Anspruch 6, worin der Wirkstoff ein biologisch aktiver Wirkstoff ist und die Zusammensetzung geeignet ist, oral an das Lebewesen verabreicht zu werden.

8. Eine Dosierungseinheitsform, umfassend
(A) eine Zusammensetzung wie in Anspruch 2 definiert; und
(B)
(a) einen Hilfsstoff,
(b) ein Streckmittel,
(c) ein Zerfallhilfsmittel,
(d) ein Gleitmittel,
(e) einen Weichmacher,
(f) ein Farbmittel,
(g) einen Dosierträger (dosing vehicle), oder
(h) jegliche Kombination derselben.

9. Die Dosierungseinheitsform gemäß Anspruch 8, umfassend eine Tablette, eine Kapsel oder eine Flüssigkeit.

10. Ein Verfahren zum Herstellen einer Zusammensetzung, umfassend das Mischen von:
(A) wenigstens einem biologisch aktiven Wirkstoff;
(B) wenigstens einer Verbindung wie in Anspruch 1 definiert; und
(C) optional einem Dosierträger.

11. Eine Verbindung gemäß Anspruch 1 der folgenden Formel 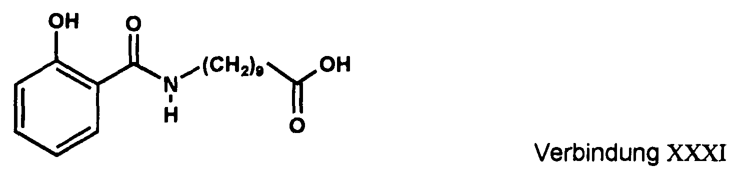 oder ein Salz derselben.

12. Eine Zusammensetzung gemäß Anspruch 3, umfassend
(a) eine Verbindung der folgenden Formel 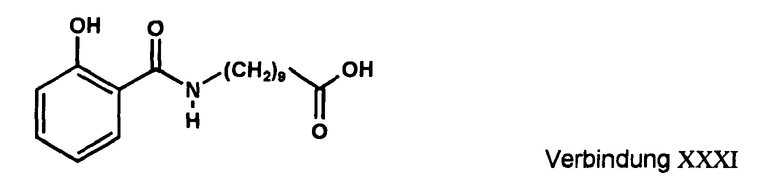 oder ein Salz derselben; und
(b) einen biologisch aktiven Wirkstoff.

13. Die Zusammensetzung gemäß Anspruch 12, worin der biologisch aktive Wirkstoff Heparin ist.

14. Eine Verbindung gemäß Anspruch 1 der folgenden Formel 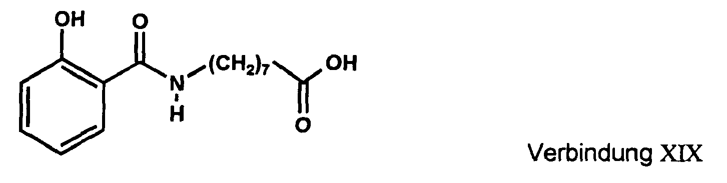 oder ein Salz derselben.

15. Eine Zusammensetzung gemäß Anspruch 3, umfassend
(a) eine Verbindung der folgenden Formel 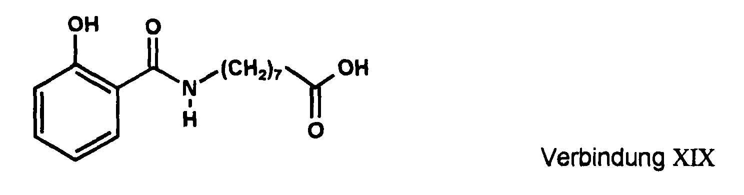 oder ein Salz derselben, und
(b) einen biologisch aktiven Wirkstoff.

16. Die Zusammensetzung des Anspruchs 15, worin der biologisch aktive Wirkstoff ein menschliches Wachstumshormon ist.

17. Die Zusammensetzung des Anspruchs 15, worin der biologisch aktive Wirkstoff Interferon ist.

18. Die Zusammensetzung des Anspruchs 15, worin der biologisch aktive Wirkstoff Calcitonin ist.

19. Die Zusammensetzung des Anspruchs 18, worin der biologisch aktive Wirkstoff Lachs-Calcitonin ist.

20. Die Zusammensetzung des Anspruchs 15, worin der biologisch aktive Wirkstoff Heparin ist.

21. Die Zusammensetzung gemäß irgendeinem der Ansprüche 12, 13, 15 oder 20, wobei die Zusammensetzung geeignet ist, oral verabreicht zu werden.

22. Eine Zusammensetzung gemäß Anspruch 3, umfassend eine Verbindung der folgenden Formel 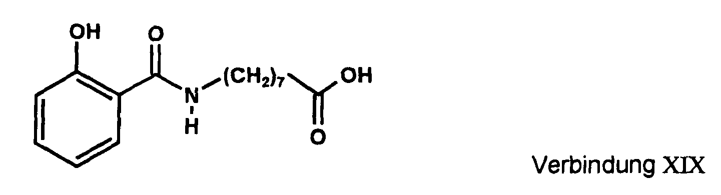 oder ein Salz derselben; und
ein Parathyroidhormon.

## Revendications

1. Composé choisi dans le groupe constitué de : 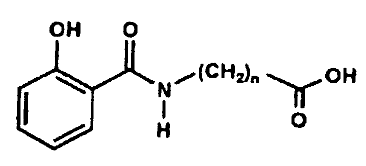 où
| Composé | n |
|---|---|
| XIX | 7 |
| XXVII | 10 |
| XXVIII | 8 |
| XXIX | 6 |
| XXX | 11 |
| XXXI | 9 |

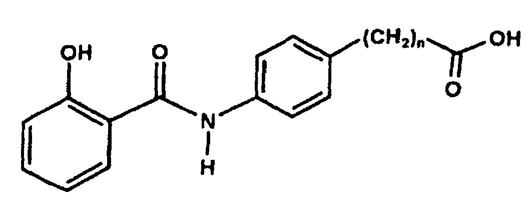
où
| Composé | n |
|---|---|
| LIV | 2 |

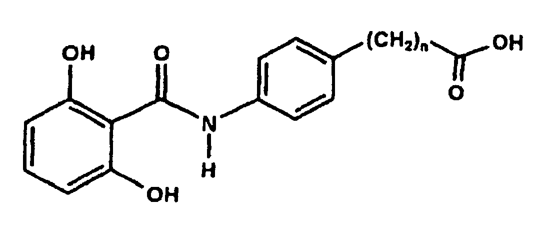
où
| Composé | n |
|---|---|
| LIII | 3 |
| LVI | 2 |

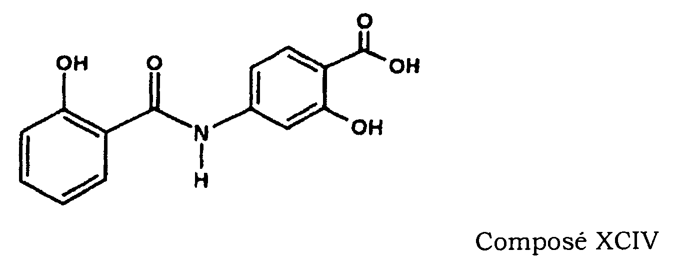

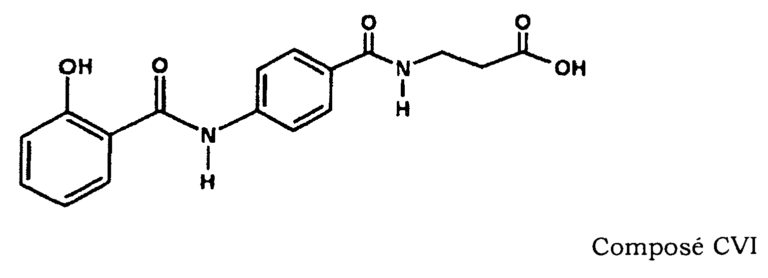

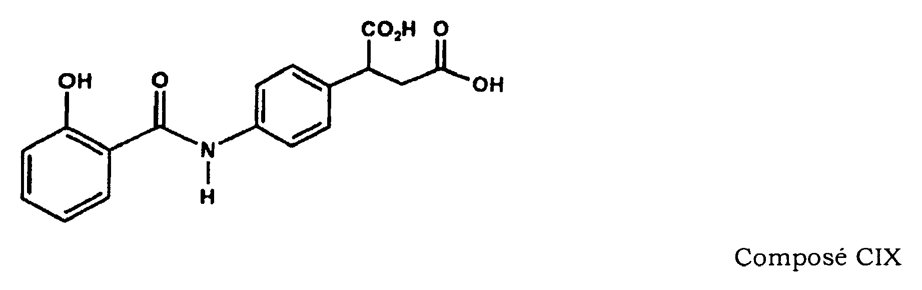
ou un sel de ceux-ci.

2. Composition comprenant :
a) un principe actif ;
b) un composé choisi dans le groupe constitué de : 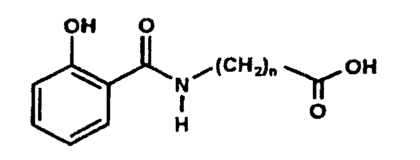
où
| Composé | n |
|---|---|
| IV | 3 |
| XIX | 7 |
| XXVII | 10 |
| XXVIII | 8 |
| XXIX | 6 |
| XXX | 11 |
| XXXI | 9 |

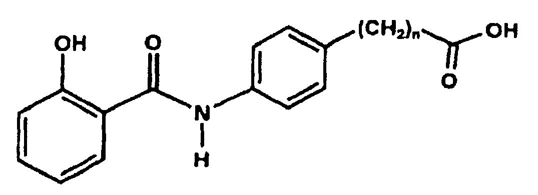
où
| Composé | n |
|---|---|
| LII | 1 |
| LIV | 2 |

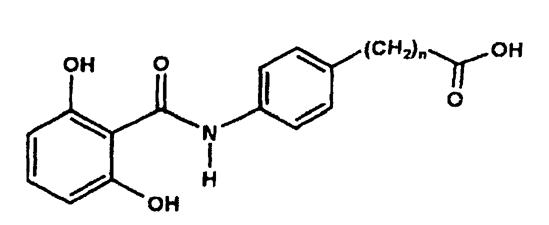
où
| Composé | n |
|---|---|
| LIII | 3 |
| LVI | 2 |

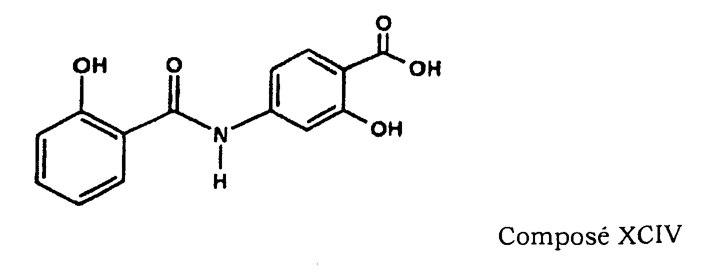

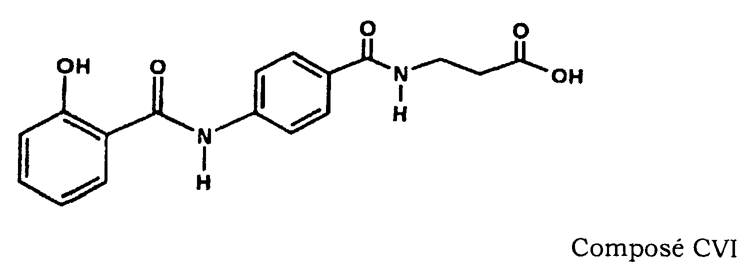

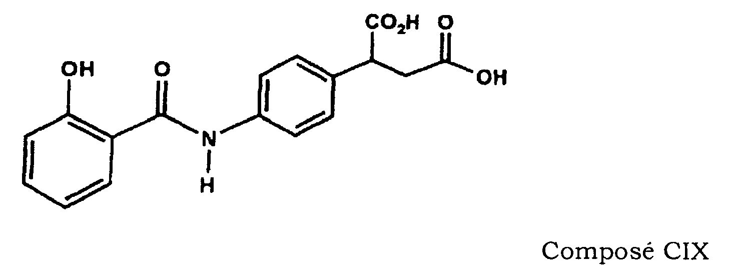
ou un sel de ceux-ci.

3. Composition selon la revendication 2, dans laquelle ledit principe actif est choisi dans le groupe constitué d'un principe biologiquement actif et d'un principe chimiquement actif.

4. Composition selon la revendication 3, dans laquelle ledit principe biologiquement actif est choisi dans le groupe constitué d'un peptide, d'un mucopolysaccharide, d'un glucide, d'un lipide, d'un pesticide ou toute combinaison de ceux-ci.

5. Composition selon la revendication 4, dans laquelle ledit principe biologiquement actif est choisi dans le groupe constitué de l'hormone de croissance humaine, de l'hormone de croissance bovine, de la somatocrinine, d'un interféron, de l'interleukine II, de l'insuline, de l'héparine, de la calcitonine, de l'érythropoïétine, du facteur natriurétique atrial, d'un antigène, d'un anticorps monoclonal, de la somatostatine, de la corticotrophine, de la gonadolibérine, de l'oxytocine, de la vasopressine, du cromoglycate de sodium, de la vancomycine, de la parathyrine, de la déféroxamine (DFO), ou toute combinaison de ceux-ci.

6. Composition selon la revendication 2, dans laquelle ladite composition est appropriée pour être administrée par voie orale, intranasale, sublinguale, intraduodénale, intramusculaire or sous-cutanée, à un animal ayant besoin d'un principe actif.

7. Composition selon la revendication 6, dans laquelle le principe actif est un principe biologiquement actif et la composition est appropriée pour être administrée par voie orale à un animal.

8. Forme posologique unitaire comprenant :
(A) une composition telle que définie dans la revendication 2 ; et
(B)
(a) un excipient,
(b) un diluant,
(c) un agent de délitement,
(d) un lubrifiant,
(e) un plastifiant,
(f) un colorant,
(g) un véhicule de dosage, ou
(h) toute combinaison de ceux-ci.

9. Forme posologique unitaire selon la revendication 8, comprenant un comprimé, une gélule ou un liquide.

10. Procédé de préparation d'une composition, ledit procédé comprenant l'etape consistant à mélanger :
(A) au moins un principe biologiquement actif ;
(B) au moins un composé tel que défini dans la revendication 1 ; et
(C) éventuellement un véhicule de dosage.

11. Composé selon la revendication 1, de formule suivante : 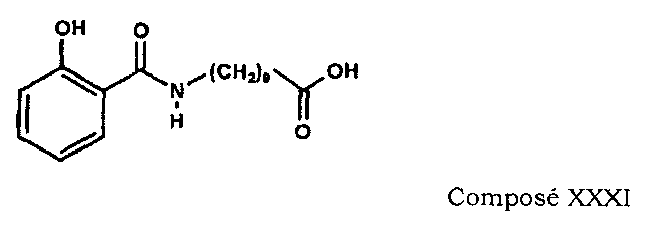 ou un sel de celui-ci.

12. Composition selon la revendication 3, comprenant :
(a) un composé de formule suivante : 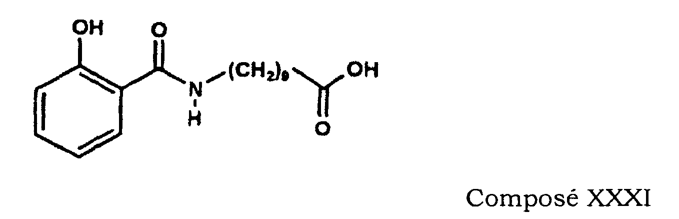 ou un sel de celui-ci ; et
(b) un principe biologiquement actif.

13. Composition selon la revendication 12, dans laquelle le principe biologiquement actif est l'héparine.

14. Composé selon la revendication 1, de formule : 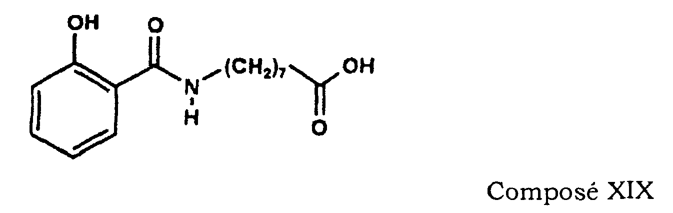 ou un sel de celui-ci.

15. Composition selon la revendication 3, comprenant :
(a) un composé de formule suivante : 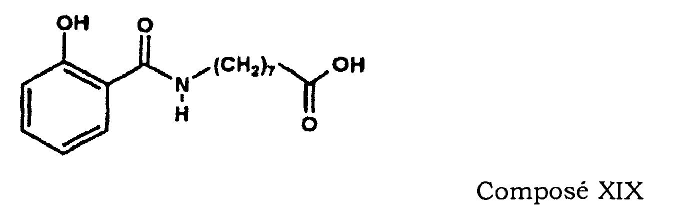 ou un sel de celui-ci ; et
(b) un principe biologiquement actif.

16. Composition selon la revendication 15, dans laquelle le principe biologiquement actif est l'hormone de croissance humaine.

17. Composition selon la revendication 15, dans laquelle le principe biologiquement actif est l'interféron.

18. Composition selon la revendication 15, dans laquelle le principe biologiquement actif est la calcitonine.

19. Composition selon la revendication 18, dans laquelle le principe biologiquement actif est la calcitonine de saumon.

20. Composition selon la revendication 15, dans laquelle le principe biologiquement actif est l'héparine.

21. Composition selon l'une quelconque des revendications 12, 13, 15 à 20, dans laquelle ladite composition est appropriée pour être administrée par voie orale.

22. Composition selon la revendication 3, comprenant un composé de formule suivante : 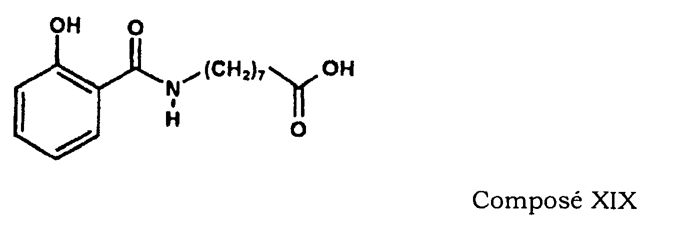 ou un sel de celui-ci ; et
et la parathyrine.
